# EUROPÄISCHE PATENTSCHRIFT

(11) **EP 0 375 612 B1**
(45) Veröffentlichungstag und Bekanntmachung des Hinweises auf die Patenterteilung: **23.08.1995**
(21) Anmeldenummer: 89810943.4
(22) Anmeldetag: 12.12.1989
(51) Int. Cl.: C07D 211/46, C08K 5/3435

(54) **Verfahren zur Herstellung von 4-Hydroxy-1,2,2,6,6-pentamethylpiperidin**
Process for the preparation of 4-hydroxy-1,2,2,6,6,-pentamethyl piperidine
Procédé pour la préparation de la 4-hydroxy-1,2,2,6,6,-pentaméthylpipéridine

(30) Priorität: 20.12.1988 CH 4763/88
(43) Veröffentlichungstag der Anmeldung: 27.06.1990
(73) Patentinhaber: CIBA-GEIGY AG, 4002 Basel (CH)
(72) Erfinder: Nitsche, Karl Stephan, Dr., D-6140 Bensheim (DE); Wolf, Walter, Dr., D-6140 Bensheim (DE); Fries, Joachim, D-6140 Bensheim (DE)

(56) Entgegenhaltungen:
- EP-A- 0 225 850
- HELVETICA CHIMICA ACTA, Band 49, Nr. 1, 31. Januar 1966, Seiten 690-695, Basel,CH; R. FRANKHAUSER et al.: "Synthese von 4-Chlorpiperidinen"

## Beschreibung

Die vorliegende Erfindung betrifft ein Verfahren zur Herstellung von 4-Hydroxy-1,2,2,6,6-pentamethylpiperidin, im folgenden auch HPMP genannt, durch katalytische Reduktion von 2,2,6,6-Tetramethyl-4-oxo-piperidin, auch Triacetonamin genannt und im folgenden mit TAA abgekürzt, anschliessende Umsetzung des entstandenen 2,2,6,6-Tetramethyl-4-hydroxy-piperidins (HTMP) mit Formaldehyd oder Paraformaldehyd und anschliessende destillative Aufarbeitung des Rohproduktes.

HPMP ist ein nützlicher Licht- und Wärmestabilisator für Kunststoffe, vor allem aber ein wertvolles Zwischenprodukt für die Synthese von einer grossen Anzahl von Lichtschutz-Additiven aus der Klasse der sterisch gehinderten Piperidinverbindungen.

HPMP wird zweckmässig durch Methylierung von HTMP hergestellt. Aus der Literatur sind für diesen Zweck verschiedene Methylierungsreaktionen bekannt, z.B. die als Eschweiler-Clarke-Reaktion bekannte Umsetzung mit Formaldehyd/Ameisensäure. Siehe dazu z.B. Helv. Chim. Acta 49(1), 690-695 (1966), Farmatsiya 13(3), 11-17 (1963), US-A 4,001,189 (Spalte 6) und US-A 3,974,127 (Spalte 5). Als Ausgangsmaterial wird dabei jeweils reines bzw. im Handel erhältliches HTMP eingesetzt. Letzteres wird nach üblichen Reduktionsmethoden, insbesondere mittels katalytischer Hydrierung, aus TAA und Isolierung des Endproduktes hergestellt (siehe z.B. CH-A 602 644). Somit war es nach dem bisherigen technischen Wissensstand erforderlich, bei der HPMP-Herstellung ausgehend von TAA, das Zwischenprodukt HTMP zu isolieren und zu reinigen.

Der hauptsächliche Nachteil dieser bekannten HPMP-Synthese-Verfahren besteht darin, dass das Zwischenprodukt, HTMP, bisher vor der Weiterreaktion grundsätzlich z.B. mittels Aussalzens oder Umkristallisierens isoliert bzw. gereinigt werden musste, wenn HPMP in einer akzeptablen Ausbeute erhalten werden soll. Die Zwischenisolierung des HTMP bringt jedoch Ausbeute-Verluste mit sich, ist zeitaufwendig und belastet die Umwelt.

In der US-A 4,731,448 ist ferner ein Verfahren zur Herstellung eines anderen Zwischenproduktes für die Herstellung von Piperidinlichtschutzmitteln, nämlich des 1-(2-Hydroxyethyl)-2,2,6,6-tetramethylpiperidins, aus TAA in 2 Stufen ohne Isolierung des Zwischenproduktes beschrieben, wobei jedoch die zweite Stufe eine völlig andersartige Reaktion, nämlich die Umsetzung mit Ethylenoxid, ist.

Es wurde nun gefunden, dass HPMP aus TAA unter Vermeidung der vorstehend angesprochenen Nachteile, insbesondere ohne Zwischenisolierung des HTMP, erhalten werden kann, wenn man die katalytische Hydrierung rein wässrig durchführt und die anfallende HTMP-Rohlösung direkt mit Formaldehyd und Ameisensäure umsetzt, wobei diese Lösung vorher vorzugsweise durch Destillation aufkonzentriert wird.

Die vorliegende Erfindung betrifft daher ein Verfahren zur Herstellung von 4-Hydroxyl-1,2,2,6,6-pentamethylpiperidin (HPMP) aus 2,2,6,6-Tetramethyl-4-oxopiperidin (TAA), das dadurch gekennzeichnet ist, dass man TAA mittels katalytischer Hydrierung in Wasser als Lösungsmittel zu 4-Hydroxy-2,2,6,6-tetramethylpiperidin (HTMP) reduziert, die anfallende HTMP-Rohlösung als solche oder nach deren Aufkonzentrierung durch Destillation, ohne Reinigung und/oder Zwischenisolierung des HTMP mit Formaldehyd oder Paraformaldehyd und Ameisensäure bei 70-150°C umsetzt, wobei Formaldehyd bzw. Paraformaldehyd in mindestens 20%igem molarem Ueberschuss und Ameisensäure in etwa stöchiometrischer Menge, jeweils bezogen auf HTMP, eingesetzt werden, die wässrige Phase des Reaktionsgemisches von der Rohproduktphase trennt und letztere destillativ aufarbeitet.

Die erste Stufe (katalytische Hydrierung) wird in Wasser als Lösungsmittel in an sich bekannter Weise unter Verwendung von üblichen Hydrierkatalysatoren, insbesondere Metall- und Edelmetallkatalysatoren, durchgeführt. Besonders geeignete Katalysatoren sind Raney-Nickel und vor allem Rutheniumkohle. Bevorzugt wird die Hydrierung in der in der CH-A 602 644 beschriebenen Weise durchgeführt.

Die nach der Hydrierung erhaltene HTMP-Lösung kann direkt als solche weiterverwendet, d.h. mit Formaldehyd bzw. Paraformaldehyd und Ameisensaure umgesetzt werden. Bevorzugt wird jedoch vor dieser Umsetzung das Volumen der Rohlösung reduziert. Dies geschieht z.B. durch Destillation unter vermindertem Druck oder vorzugsweise Normaldruck. In einer bevorzugten Ausführungsform destilliert man mindestens 10 %, insbesondere mindestens 20 %, z.B. mindestens 30 % der HTMP-Rohlösung ab. So kann man etwa 10 bis 95 %, z.B. 10 bis 80 %, vorzugsweise 10-60 %, bzw. 10-50 %, beispielsweise 10-40 % bzw. 20-50 %, vor allem 20-40 % davon abdestillieren. Man kann aber auch die Rohlösung soweit einengen, dass nur eine HTMP-Rohschmelze übrig bleibt, die dann mit Formaldehyd bzw. Paraformaldehyd und Ameisensäure direkt umgesetzt wird.

In einer weiteren möglichen Ausführungsform des erfindungsgemässen Verfahrens wird die HTMP-Rohlösung auf einen HTMP-Gehalt von mindestens 20 %, vorzugsweise mindestens 30 %, insbesondere mindestens 40 % eingeengt. So kann nach der Destillation die Rohlösung z.B. einen HTMP-Gehalt von 20-90 %, insbesondere 30-90 %, vor allem 40-90 %, z.B. 50-90 %, aufweisen.

Die Destillation der HTMP-Rohlösung kann bei Normaldruck durchgeführt werden. Wird bei vermindertem Druck destilliert, ist ein solcher von 100 bis 900, insbesondere von 100 bis 300 mbar zweckmässig. Je nach gewähltem Druck geht bei Temperaturen von beispielsweise 30-100°C, insbesondere 50 bis 100°C, ein zweiphasiges Gemisch über, das verworfen wird.

Die weitere Umsetzung der gegebenenfalls eingeengten HTMP-Rohlösung erfolgt bei 70-150°C. Bevorzugt wird die Umsetzung bei 70-120°C, insbesondere bei 70-100°C, durchgeführt, da in letzterem Fall bei Normaldruck gearbeitet werden kann. Bei Temperaturen über 100°C wird in einer Druckapparatur (z.B. in einem Autoklaven) gearbeitet. Beträgt die Reaktions-Temperatur z.B. 120°C, stellt sich ein Druck von etwa 2,5 bis 3 bar ein. Besonders bevorzugt beträgt die Reaktionstemperatur 85-100°C.

Formaldehyd bzw. Paraformaldehyd wird in mindestens 20%igem molaren Ueberschuss, bezogen auf HTMP, eingesetzt. Zweckmässig ist ein solcher von mindestens 40 %. Eine technisch bedingte Obergrenze für diesen Ueberschuss gibt es nicht, sie ergibt sich lediglich aus praktischen wirtschaftlichen Ueberlegungen. Zweckmässige molare Ueberschüsse liegen in der Regel im Bereich von 20 bis 100 %, vorzugsweise 40 bis 100 %, insbesondere 40 bis 70 %, z.B. etwa 60 %.

Formaldehyd wird zweckmässig in Form einer wässrigen Lösung, beispielsweise als 27-50%ige, vorzugsweise als 37%ige Formalinlösung eingesetzt.

Ameisensäure wird in ungefähr stöchiometrischer Menge (bezogen auf HTMP) eingesetzt, wobei ein geringer Ueberschuss die Reaktion selbstverständlich nicht stört.

Nach beendeter Reaktion ist das Reaktionsgemisch zweiphasig; es besteht aus einer oberen Produktphase (Rohproduktphase, "Rohschmelze") und einer unteren wässrigen Phase. Zur weiteren Aufarbeitung wir letztere von der HPMP-Rohschmelze abgetrennt. Dies geschieht z.B. zweckmässig durch Ablassen der wässrigen Phase durch den Boden des Reaktionsgefässes. Die Rohschmelze wird destillativ aufgearbeitet. Zu diesem Zweck werden vorteilhaft in einer üblichen Destillationsapparatur zunächst niedrig siedende Nebenprodukte abdestilliert, vorzugsweise bei vermindertem Druck, z.B. in einem Vakuum von 1000-30 mbar. Dabei werden insbesondere leicht-flüchtige Anteile mit einem Siedepunkt < 150°C entfernt. Danach ist es zweckmässig, aber nicht Bedingung, bei etwa 30-10 mbar und ca. 80-110°C, z.B. 90-100°C, höher siedende Nebenprodukte abzuziehen. Die Destillation des HPMP selbst erfolgt vorzugsweise bei 10-2 mbar und einer Kopftemperatur von 112°C.

In einer alternativen Ausführungsform kann vor der Phasentrennung dem Reaktionsgemisch ein organisches, mit Wasser schwer mischbares Lösungsmittel zugesetzt werden, in dem sich HPMP löst. Letzteres geht dann in die organische Phase, mit der es Von der wässrigen Phase abgetrennt werden kann. Das organische Lösungsmittel wird dann durch Destillation entfernt und die zurückbleibende HPMP-Rohschmelze wird destillativ aufgearbeitet, z.B. wie im vorstehenden Absatz beschrieben. Als organische Lösungsmittel kommen beispielsweise aliphatische, cycloaliphatische oder aromatische Kohlenwasserstoffe zum Einsatz. Beispiele hiefür sind verschiedene Benzinfraktionen, Benzol, Toluol und Xylol.

Das HPMP-Destillat kann gegebenenfalls anschliessend nach üblichen Verfahren granuliert werden, um eine besser lagerfähige und leichter handhabbare Form zu erhalten.

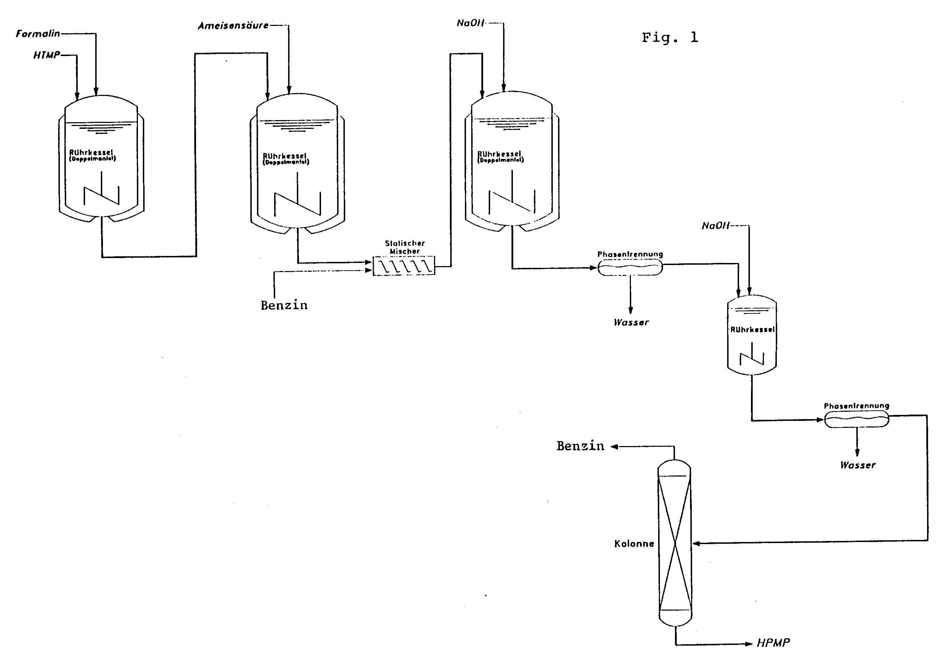

Vor der Aufarbeitung der Reaktionsmischung, d.h. vor der Abtrennung der Rohproduktphase bzw. vor oder nach Zugabe des organischen Lösungsmittels, ist es zweckmässig, überschüssiges Formaldehyd durch Zugabe einer Base, z.B. eines organischen Amins oder vorzugsweise eines Alkali- oder Erdalkalimetallhydroxids, insbesondere von KOH oder NaOH, zu zerstören. Die dazu verwendete Menge soll ausreichen, den eingesetzten Formaldehydüberschuss zu beseitigen. Vorzugsweise setzt man mindestens 30 % mehr Base ein, als theoretisch zur Zerstörung des eingesetzten stöchiometrischen Ueberschusses an Formaldehyd nötig wäre. Das erfindungsgemässe Verfahren kann diskontinuierlich (batchwise) oder kontinuierlich erfolgen. Besonders die zweite Stufe kann mit Vorteil kontinuierlich durchgeführt werden, wobei man die in der Verfahrenstechnik üblichen Vorrichtungen benutzen kann. In Frage kommen beispielsweise ein Rotating-Disc-Reaktor oder eine Reaktionskaskade. Ein Beispiel für eine mögliche Anordnung für die kontinuierliche Durchführung des erfindungsgemässen Verfahrens ist der Fig. 1 zu entnehmen.

Für die Bestimmung des Gehaltes an HTMP in der Rohlösung und an HPMP in der Endprodukt-Lösung können übliche analytische Methoden verwendet werden, wobei die Gehaltsbestimmung vorzugsweise gaschromatographisch erfolgt.

Mit dem erfindungsgemässen Verfahren gelingt es, HPMP aus TAA ohne Zwischenisolierung des HTMP in hohen Ausbeuten zu erhalten. Das Verfahren ist einfach durchzuführen, zeitsparend und darüber hinaus ökologisch günstig, da die bisher beim Aussalzen bzw. Umkristallisieren anfallenden Salz- bzw. Lösungsmittelmengen vermieden werden. Eine weitere günstige Folge des erfindungsgemässen Verfahrens ist die höhere Raum-Zeit-Ausbeute und geringere Herstellungskosten.

Es ist als besonders überraschend anzusehen, dass die Methylierung des HTMP trotz des durch das Weglassen der Isolierung des letzteren hohen Nebenproduktspiegels eine praktisch 100%ige Umsetzung erzielt, dass die vollständige Umsetzung in kürzerer Zeit beendet ist und dass sogar eine bessere Qualität des Endproduktes erreicht wird. Ebenso überraschend ist es, dass die Selektivität bei hoher Temperatur (z.B. 100°C) ebenso gut ist wie bei tieferer Temperatur (z.B. 70°C).

Ferner kann im erfindungsgemässen Verfahren der Ueberschuss an Formaldehyd geringer gehalten werden als bei den konventionellen Verfahren. Ausserdem gelingt eine vollständige Eliminierung des gebildeten CO₂.

Die folgenden Beispiele erläutern die Erfindung näher. Teile und Prozente beziehen sich darin sowie in der gesamten übrigen Beschreibung und in den Patentansprüchen auf das Gewicht, sofern nichts anderes angegeben ist. Die Gehaltsbestimmung an HTMP und HPMP in den Beispielen erfolgt gaschromatographisch.

Beispiel 1: 1800 kg destilliertes TAA (Reinheitsgrad 92-98 %) werden mit 2200 Liter Wasser verdünnt und bei 70° bis 80°C und 10 bar Wasserstoffdruck in Gegenwart von Rutheniumkohle hydriert. Der Katalysator wird anschliessend abfiltriert und die HTMP-Rohlösung vor der Weiterverarbeitung in einem Tank aufbewahrt.
HTMP-Gehalt der Lösung: 40 %, Ausbeute 91-97 %

Beispiel 2: 415 g der gemäss Beispiel 1 erhaltenen HTMP-Rohlösung werden vorgelegt und bei 60-100°C 37 % davon azeotrop abdestilliert. Das Destillat fällt zweiphasig an. Der verbleibende Destillationsrückstand (216 g; HTMP-Gehalt ca. 60 %) wird zusammen mit 50 g Paraformaldehyd in einem Reaktionsgefäss vorgelegt, auf 86°C erhitzt und anschliessend innerhalb von 20 Minuten unter Rühren mit 57 g Ameisensäure (als 85%ige wässrige Lösung) versetzt. Danach erhitzt man auf Rückflusstemperatur und rührt bis zu einer Gesamtreaktionszeit von 3 Stunden. Danach wird das Reaktionsgemisch mit 85 g 50%iger wässriger NaOH versetzt und abkühlen gelassen. Die obere, ölige Produktphase wird abgetrennt und in einer üblichen Destillationsapparatur destilliert. Zunächst werden bei einer Kopftemperatur von 40°C und einem Vakuum von 1000-30 mbar die leichtflüchtigen Anteile mit einem Siedepunkt von < 150°C abgezogen. Anschliessend wird bei 30-10 mbar und einer Kopftemperatur von 100°C der Vorlauf abgenommen. Danach destilliert man bei 10-2 mbar und einer Kopftemperatur von 112°C die Hauptfraktion des HPMP über, welches nach dem Abkühlen in Form weisser Kristalle mit einem Schmelzpunkt von 76°C anfällt. Ausbeute nach der Destillation: 89 % der Theorie, bezogen auf eingesetztes HTMP. Rohausbeute vor der Destillation: 99-100 %.

Das geschmolzene Produkt kann durch Granulierung in eine vorteilhaftere Lagerform übergeführt werden. Dies geschieht auf einem Kühlband oder einer Kühlwalze.

Beispiel 3: 422,6 g der gemäss Beispiel 1 erhaltenen HTMP-Rohlösung werden vorgelegt und bei 60-100°C 47 % davon azeotrop abdestilliert. Das Destillat fällt zweiphasig an. Der verbleibende Destillationsrückstand (224 g, HTMP-Gehalt ca. 70 %) wird zusammen mit 135 g 37%iger Formalinlösung in einem Reaktionsgefäss vorgelegt, auf 86°C erhitzt und anschliessend innerhalb von 20 Minuten unter Rühren mit 57 g Ameisensaure (als 85%ige wässrige Lösung) versetzt. Danach erhitzt man auf Rückflusstemperatur und rührt bis zu einer Gesamtreaktionszeit von 3 Stunden. Danach wird das Reaktionsgemisch mit 85 g 50%iger wässriger NaOH versetzt und abkühlen gelassen. Die weitere Aufarbeitung erfolgt genau wie in Beispiel 2 beschrieben. Ausbeute und Schmelzpunkt des HPMP sind die gleichen wie in Beispiel 2.

Beispiel 4: Beispiel 3 wird wiederholt, jedoch an Stelle von 135 g Formalinlösung 50 g Paraformaldehyd eingesetzt. Man erhält HPMP in gleicher Reinheit und Ausbeute wie in Beispiel 3 bzw. 2.

Beispiel 5: 417 g der gemäss Beispiel 1 hergestellten HTMP-Rohlösung werden vorgelegt und bei 60-100°C 58 % davon azeotrop abdestilliert. Das Destillat fällt zweiphasig an. Der verbleibende Destillationsrückstand (175,4 g; HTMP-Gehalt ca. 89 %) wird zusammen mit 42 g Paraformaldehyd in einem Reaktionsgefäss vorgelegt, auf 86°C erhitzt und anschliessend unter Rühren innerhalb von 20 Minuten mit 57 g Ameisensäure (als 85%ige wässrige Lösung) versetzt. Danach erhitzt man auf Rückflusstemperatur und rührt bis zu einer Gesamtreaktionszeit von 3 Stunden. Danach wird das Reaktionsgemisch mit 85 g 50%iger wässriger NaOH versetzt und abkühlen gelassen. Die weitere Aufarbeitung erfolgt genau wie in Beispiel 2 beschrieben. Ausbeute und Schmelzpunkt des HPMP sind die gleichen wie in Beispiel 2.

Beispiel 6: In einem Mischkessel werden 100 Teile der gemäss Beispiel 1 erhaltenen HTMP-Rohlösung in 85 Teilen 37%iger Formalinlösung gelöst. Diese Mischung wird auf 80°C erwärmt und mittels Dosierpumpe in einen Reaktor gefördert, wo sie mit 36 Teilen Ameisensäure (als 85%ige wässrige Lösung) mit einer Dosiergeschwindigkeit so versetzt wird, dass die Temperatur nicht über 86°C steigt. Nach einer Verweilzeit von 2 Stunden pumpt man die Lösung über einen statischen Mischer, wo sie mit einer Benzinfraktion (Siedebereich 100-140°C) vermischt wird, in einen weiteren Rührkessel, wo die Reaktionslösung mit 30 Teilen NaOH extrahiert wird. In einem nachgeschalteten flüssig/flüssig-Separator erfolgt die Abtrennung der wässrigen Phase. In einer zweiten Extraktionseinheit wird nochmals mit NaOH extrahiert. Zuletzt wird das Benzin in einer Destillationskolonne abdestilliert und die Rohschmelze am Boden kontinuierlich abgezogen. Die Rohschmelze wird dann in einer Destillationsapparatur in der in Beispiel 2 beschriebenen Weise destillativ gereinigt. Reines HPMP wird auf diese Weise in der in Beispiel 2 angegebenen Ausbeute und Reinheit erhalten. Der Granulierungsschritt wird vorzugsweise ebenfalls angeschlossen.

Fig. 1 zeigt eine schematische Darstellung der verwendeten kontinuierlichen Reaktionsanlage.

## Patentansprüche

1. Verfahren zur Herstellung von 4-Hydroxy-1,2,2,6,6-pentamethylpiperidin (HPMP) aus 2,2,6,6-Tetramethyl-4-oxopiperidin (TAA), dadurch gekennzeichnet, dass man TAA mittels katalytischer Hydrierung in Wasser als Lösungsmittel zu 4-Hydroxy-2,2,6,6-tetramethylpiperidin (HTMP) reduziert, die anfallende HTMP-Rohlösung als solche oder nach deren Aufkonzentrierung durch Destillation, ohne Reinigung und/oder Zwischenisolierung des HTMP mit Formaldehyd oder Paraformaldehyd und Ameisensäure bei 70-150°C umsetzt, wobei Formaldehyd bzw. Paraformaldehyd in mindestens 20%igem molaren Ueberschuss und Ameisensäure in etwa stöchiometrischer Menge, jeweils bezogen auf HTMP, eingesetzt wird, die wässrige Phase des Reaktionsgemisches von der Rohproduktphase trennt und letztere destillativ aufarbeitet.

2. Verfahren nach Anspruch 1, worin man die HTMP-Rohlösung durch Destillation einengt.

3. Verfahren nach Anspruch 2, worin man mindestens 10 %, vorzugsweise mindestens 30 %, der HTMP-Rohlösung abdestilliert.

4. Verfahren nach Anspruch 2, worin man die HTMP-Rohlösung bis zu einem HTMP-Gehalt von mindestens 20 %, vorzugsweise mindestens 50 %, aufkonzentriert.

5. Verfahren nach Anspruch 2, worin man bis zu einer HTMP-Rohschmelze aufkonzentriert.

6. Verfahren nach Anspruch 1, worin man die HTMP-Lösung ohne Destillation weiterverarbeitet.

7. Verfahren nach Anspruch 1, worin die Umsetzung der HTMP-Rohlösung kontinuierlich durchgeführt wird.

8. Verfahren nach Anspruch 1, worin die Umsetzung der HTMP-Rohlösung diskontinuierlich durchgeführt wird.

9. Verfahren nach Anspruch 1, worin die Umsetzung der HTMP-Rohlösung bei 70 bis 100°C, insbesondere 85 bis 100°C, erfolgt.

10. Verfahren nach Anspruch 1, worin die HTMP-Rohlösung mit Paraformaldehyd oder 27-50%iger Formalinlösung umgesetzt wird.

11. Verfahren nach Anspruch 1, worin der molare Ueberschuss an Formaldehyd bzw. Paraformaldehyd mindestens 40 %, insbesondere 40-70 % beträgt.

12. Verfahren nach Anspruch 1, worin nach der destillativen Aufarbeitung das destillierte Endprodukt HPMP granuliert wird.

13. Verfahren nach Anspruch 1, worin der Reaktionsmischung vor Abtrennung der Rohproduktphase ein mit Wasser schwer mischbares organisches Lösungsmittel zugesetzt wird, worin sich HPMP löst und diese organische Phase von der wässrigen Phase abgetrennt wird.

14. Verfahren nach Anspruch 1 oder 13, worin vor der Abtrennung der Rohproduktphase bzw. vor oder nach Zugabe des organischen Lösungsmittels in der Reaktionsmischung überschüssiges Formaldehyd durch Zugabe einer Base, vorzugsweise KOH oder NaOH, zerstört wird.

15. Verfahren nach Anspruch 13, worin als organisches Lösungsmittel ein aliphatischer oder aromatischer Kohlenwasserstoff verwendet wird.

## Claims

1. A process for the preparation of 4-hydroxyl-1,2,2,6,6-pentamethylpiperidine (HPMP) from 4-oxo₋2,2,6,6-tetramethylpiperidine (TAA), which comprises reducing TAA to 4-hydroxy-2,2,6,6-tetramethylpiperidine (HTMP) by means of catalytic hydrogenation in water as solvent, reacting the resulting crude HTMP solution, as obtained or after concentration by distillation, with formaldehyde or paraformaldehyde and formic acid at 70-150°C, without purification and/or isolation of HTMP, using formaldehyde or paraformaldehyde in at least 20 % molar excess and formic acid in about the stoichiometric amount, based in each case on HTMP, separating the aqueous phase of the reaction mixture from the crude product phase and working up said product phase by distillation.

2. A process according to claim 1, wherein the crude HTMP solution is concentrated by distillation.

3. A process according to claim 2, wherein at least 10 %, preferably at least 30 %, of the crude HTMP solution is distilled off.

4. A process according to claim 2, wherein the crude HTMP solution is concentrated to an HTMP content of at least 20 %, preferably at least 50 %.

5. A process according to claim 2, wherein the solution is concentrated to a crude HTMP melt.

6. A process according to claim 1, wherein the HTMP solution is processed further without distillation.

7. A process according to claim 1, wherein the crude HTMP solution is reacted continuously.

8. A process according to claim 1, wherein the crude HTMP solution is reacted batchwise.

9. A process according to claim 1, wherein the crude HTMP solution is reacted at 70 to 100°C, especially 85 to 100°C.

10. A process according to claim 1, wherein the crude HTMP solution is reacted with paraformaldehyde or 27-50 % strength formalin solution.

11. A process according to claim 1, wherein the molar excess of formaldehyde or paraformaldehyde is at least 40 %, especially 40-70 %.

12. A process according to claim 1, wherein after working-up by distillation the distilled HPMP final product is granulated.

13. A process according to claim 1, wherein an organic solvent which is sparingly miscible with water, and in which HPMP dissolves, is added to the reaction mixture before separation of the crude product phase, and this organic phase is separated from the aqueous phase.

14. A process according to claim 1 or claim 13, wherein before separation of the crude product phase or before or after addition of the organic solvent, excess formaldehyde in the reaction mixture is destroyed by the addition of a base, preferably KOH or NaOH.

15. A process according to claim 13, wherein an aliphatic or aromatic hydrocarbon is used as organic solvent.

## Revendications

1. Procédé pour la préparation de la 4-hydroxy-1,2,2,6,6-pentaméthylpipéridine (HPMP) à partir de la 2,2,6,6-tétraméthyl-4-oxopipéridine (TAA) caractérisé en ce qu'on réduit la TAA par hydrogénation catalytique dans l'eau en tant que solvant pour obtenir la 4-hydroxy-2,2,6,6-tétraméthylpipéridine, on fait réagir la solution brute de HTMP formée telle que ou après concentration par distillation, sans purification et/ou isolement intermédiaire de la HTMP, avec le formaldéhyde ou le paraformaldéhyde et l'acide formique à une température de 70 à 150°C, en utilisant le formaldéhyde, respectivement le paraformaldéhyde en un excès d'au moins 20% en moles et l'acide formique en quantité environ stoechiométrique, chaque fois par rapport à la HTMP, on sépare la phase aqueuse du mélange réactionnel de la phase de produit brut et on traite cette dernière par distillation.

2. Procédé selon la revendication 1, où on concentre la solution brute de HTMP par distillation.

3. Procédé selon la revendication 2, où on sépare par distillation d'au moins 10%, de préférence d'au moins 30% de la solution brute de HTMP.

4. Procédé selon la revendication 1, où on concentre la solution brute de HTMP jusqu'à une teneur en HTMP d'au moins 20%, de préférence d'au moins 50%.

5. Procédé selon la revendication 2, où on concentre jusqu'à l'obtention d'une fonte brute de HTMP.

6. Procédé selon la revendication 1, où on effectue le traitement complémentaire de la solution HTMP sans distillation.

7. Procédé selon la revendication 1, où on met en oeuvre la réaction de la solution brute de HTMP en continu.

8. Procédé selon la revendication 1, où on met en oeuvre la réaction de la solution brute de HTMP en discontinu.

9. Procédé selon la revendication 1, où on effectue la réaction de la solution brute de HTMP à une température de 70 à 100°C, plus particulièrement de 85 à 100°C.

10. Procédé selon la revendication 1, où on fait réagir la solution brute de HTMP avec le paraformaldéhyde ou une solution de formaline à 25 à 50%.

11. Procédé selon la revendication 1, où l'excès en moles en formaldéhyde, respectivement en paraformaldéhyde est d'au moins 40%, plus particulièrement de 40 à 70%.

12. Procédé selon la revendication 1, où après traitement complémentaire par distillation, on granule le produit final distillé, la HPMP.

13. Procédé selon la revendication 1, où on ajoute au mélange réactionnel avant la séparation de la phase du produit brut, un solvant organique difficilement miscible à l'eau, dans lequel la HPMP se dissout et on sépare cette phase organique de la phase aqueuse.

14. Procédé selon la revendication 1 ou 13, où avant la séparation de la phase de produit brut, respectivement avant ou après l'addition du solvant organique au mélange réactionnel on détruit l'excès en formaldéhyde par addition d'une base, de préférence de KOH ou de NaOH.

15. Procédé selon la revendication 13, où on utilise comme solvant organique un hydrocarbure aliphatique ou aromatique.
